(19) Europäisches Patentamt
European Patent Office
Office européen des brevets

(11) Veröffentlichungsnummer: **0 127 063**
**B1**

(12) # EUROPÄISCHE PATENTSCHRIFT

(45) Veröffentlichungstag der Patentschrift: **13.07.88**

(51) Int. Cl.⁴: **G 03 C 7/26,** G 03 C 7/32 // C07D257/04

(21) Anmeldenummer: **84105496.8**

(22) Anmeldetag: **15.05.84**

(54) **Fotografisches Aufzeichnungsmaterial mit einer Vorläuferverbindung einer fotografisch wirksamen Verbindung.**

(30) Priorität: **28.05.83 DE 3319428**

(43) Veröffentlichungstag der Anmeldung: **05.12.84 Patentblatt 84/49**

(45) Bekanntmachung des Hinweises auf die Patenterteilung: **13.07.88 Patentblatt 88/28**

(84) Benannte Vertragsstaaten: **BE DE FR GB IT**

(56) Entgegenhaltungen:
**DE - A - 2 640 601**
**DE - A - 2 716 204**

(73) Patentinhaber: **Agfa-Gevaert AG, Patentabteilung, D-5090 Leverkusen 1 (DE)**

(72) Erfinder: **Bergthaller, Peter, Dr., Leuchter Gemark 5a, D-5060 Bergisch Gladbach 2 (DE)**
Erfinder: **Odenwälder, Heinrich, Dr., Am Arenzberg 37, D-5090 Leverkusen 3 (DE)**
Erfinder: **Matejec, Reinhard, Dr., Hegelstrasse 25, D-5090 Leverkusen 1 (DE)**

**EP 0 127 063 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein fotografisches Aufzeichnungsmaterial mit mindestens einer lichtempfindlichen Silberhalogenidemulsionsschicht und mindestens einer Vorläuferverbindung einer fotografisch wirksamen Verbindung, aus der die fotografisch wirksame Verbindung unter den Bedingungen der fotografischen Entwicklung in bildmässiger Verteilung freigesetzt wird.

Die bildmässige Freisetzung fotografisch wirksamer Verbindungen ist seit geraumer Zeit eine dem Fachmann geläufige Technik. Man macht hiervon beispielsweise Gebrauch bei verschiedenen Farbdiffusionsübertragungsverfahren, bei denen eine fotografisch wirksame Verbindung in diesem Fall ein diffusionsfähiger Farbstoff, als Folge der Entwicklung von bildmässig belichtetem Silberhalogenid aus einem Farbabspalter bildmässig in Freiheit gesetzt und auf eine Bildempfangsschicht übertragen wird. Die vorliegende Erfindung befasst sich mit einer solchen Art von Vorläuferverbindungen, die die fotografisch wirksame Verbindung als Folge einer durch die Entwicklung von bildmässig belichtetem Silberhalogenid ausgelösten oxidativen Kupplungsreaktion bildmässig freisetzen. Ein auf einer solchen Reaktion beruhendes Farbdiffusionsübertragungsverfahren ist beispielsweise in DE-C-1 095 115 beschrieben. Bei einer Variante des hier beschriebenen Verfahrens werden Farbkupplerverbindungen verwendet, die in der Kupplungsstelle einen farbigen Molekülteil enthalten und diesen als diffusionsfähigen Farbstoff freisetzen, wenn der kupplungsfähige Teil der Verbindung mit dem Oxidationsprodukt einer Farbentwicklerverbindung, nämlich einer p-Phenylendiaminverbindung, reagiert. Anstelle eines Farbstoffrestes kann auf diese Weise auch eine andere Art von fotografisch wirksamen Verbindungen freigesetzt werden, z.B. ein Kuppler, ein Härtungsmittel, ein Silberhalogenidlösungsmittel oder eine sonstige Verbindung, die auf bei der Entwicklung und Verarbeitung des fotografischen Aufzeichnungsmaterials ablaufende Reaktionen beschleunigend oder verzögernd Einfluss zu nehmen vermag. Besondere Bedeutung in dieser Hinsicht haben die sogenannten DIR-Kuppler erlangt; dies sind Kuppler, die bei der Kupplung einen an die Kupplungsstelle gebundenen Entwicklungsinhibitor freisetzen (DIR = development inhibitor releasing). Bezüglich der DIR-Kuppler sei auf US-A-3 227 554 verwiesen. Auch aus Verbindungen, die unter den Bedingungen der fotografischen Entwicklung zur oxidativen Kupplung befähigt sind, ohne hierbei durch die Kupplung bedingt einen Farbstoff zu erzeugen, können fotografisch wirksame Verbindungen freigesetzt werden; zu verweisen ist beispielsweise auf DE-C-1 547 640. Die Verwendung von DIR-Kupplern ist beispielsweise von Bedeutung für verschiedene fotografische Effekte, wie Interimage-Effect, Gradationsbeeinflussung und Schärfeverbesserung [Photographic Science and Engineering 13, 74 (1969)].

Eine deutliche weitere Verbesserung kann erzielt werden, wenn die fotografisch wirksame Verbindung nicht bereits unmittelbar nach der die Abspaltung aus dem Kuppler auslösenden Kupplungsreaktion wirksam wird, sondern erst mit einer gewissen zeitlichen Verzögerung danach. Dies bedeutet, dass die fotografisch wirksame Verbindung oder eine Vorstufe davon nach der Abspaltung von dem Kupplerteil während einer gewissen Zeitspanne nach der Abspaltung aufgrund ihrer Diffusionsfähigkeit in eine gewisse räumliche Umgebung vom Ort der Abspaltung zu diffundieren vermag, bevor sie wirksam wird. Das Wirksamwerden der fotografisch wirksamen Verbindung kann erreicht werden durch eine zeitlich gesteuerte Abspaltung eines sogenannten Zeitsteuergliedes, an das die fotografische wirksame Verbindung gebunden ist und das seinerseits an die Kupplungsstelle des Kupplers gebunden ist. Die Abspaltung der fotografisch wirksamen Verbindung von dem Zeitsteuerglied ist eine Folge der Abspaltung des Zeitsteuergliedes mit der daran gebundenen fotografisch wirksamen Verbindung von dem Kuppler. Vorläuferverbindungen dieser Art sind beispielsweise bekannt aus DE-A-27 03 145, DE-A-28 55 697, DE-A-31 05 026 und DE-A-32 09 671. Bekannte Zeitsteuerglieder sind demnach beispielsweise

eine Gruppe

R
|
–O–CH–,

wobei das O-Atom an die Kupplungsstelle des Kupplers und das C-Atom an ein N-Atom einer fotografisch wirksamen Verbindung gebunden ist,

eine Gruppe, die nach Abspaltung vom Kuppler einer intramolekularen nukleophilen Verdrängungsreaktion unterliegt und hierbei die fotografisch wirksame Verbindung zugänglich macht, oder

eine Gruppe, in der eine Elektronenübertragung entlang eines konjugierten Systems stattfinden kann, wodurch die fotografisch wirksame Verbindung abgespalten wird.

Zwar konnten mit Hilfe der bekannten DIR-Kuppler mit oder ohne Zeitsteuerglied einige der für die Bildqualität wichtigen Eigenschaften deutlich verbessert werden. Es besteht jedoch weiterhin ein Bedarf an neuen Vorläuferverbindungen für fotografisch wirksame Verbindungen, aus denen die fotografisch wirksamen Verbindungen zeitlich gesteuert in Freiheit gesetzt werden, und mit denen einige wichtige Eigenschaften, z.B. Interimageeffekte und Kanteneffekte weiter verbessert werden können. Es wurden nun Vorläuferverbindungen mit einer neuartigen Zeitsteuergruppe gefunden.

Gegenstand der Erfindung ist ein fotografisches Aufzeichnungsmaterial mit mindestens einer Silberhalogenidemulsionsschicht, gekennzeichnet durch den Gehalt an einer Verbindung der folgenden Formel I

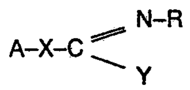 I

worin bedeuten

A den Rest einer Verbindung, die unter den Bedingungen der fotografischen Entwicklung zur oxidativen Kupplung mit einer Silberhalogenidentwicklerverbindung befähigt ist;

X –O–, –S– oder $-NR^1-$;

Y einen abspaltbaren Rest, der unter den Bedingungen der fotografischen Entwicklung unter Mitnahme des Bindungselektronenpaares zwischen C und Y von C abgespalten wird, wenn X unter Mitnahme des Bindungselektronenpaares zwischen A und X von A abgespalten wird, und der der Rest einer fotografisch wirksamen Verbindung ist oder der Rest einer alkalilabilen Vorläuferverbindung einer fotografisch wirksamen Verbindung ist;

R Alkyl, Aralkyl, Aryl, Acyl, eine heterocyclische Gruppe oder eine Gruppe –NH-Acyl, $-OR^2$ oder $-P(O)(OR^2)_2$;

$R^1$ H, Alkyl, Aryl, Alkylsulfonyl oder Arylsulfonyl; und

$R^2$ Alkyl, Aryl oder eine heterocyclische Gruppe.

A ist vorzugsweise der Rest eines Farbkupplers oder der Rest einer im wesentlichen nicht farbig kuppelnden kupplungsfähigen Verbindung und die Gruppe

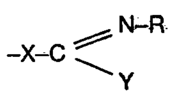

ist an die Kupplungsstelle von A gebunden. Als Farbkuppler ist A beispielsweise der Rest eines üblichen Blaugrünkupplers, Purpurkupplers oder Gelbkupplers. Die hierfür in Frage kommenden Strukturen sind dem Fachmann geläufig. Wenn A der Rest eines Farbkupplers ist, handelt es sich bei den Verbindungen der vorliegenden Erfindung in der Regel um 2-Äquivalentkuppler. In der Regel ist A mit Ballastgruppen versehen, um eine diffusionsfeste Einlagerung der erfindungsgemässen Vorläuferverbindungen in fotografische Schichten zu ermöglichen. Es können jedoch auch solche Vorläuferverbindungen in Frage kommen, die als vollständige Verbindung eine gewisse, wenn auch beschränkte Diffusionsfähigkeit haben.

Der abspaltbare Rest Y ist in der Regel über ein Heteroatom an C gebunden, wobei dieses Heteroatom Bestandteil des abspaltbaren Restes ist. So kann der Rest der fotografisch wirksamen Verbindung mit einem Sauerstoff-, Schwefel-, Selen- oder Stickstoffatom oder z.B. mit einer $SO_2$-Gruppe an das in Formel I dargestellte C-Atom gebunden sein. Die fotografisch wirksame Verbindung ist beispielsweise ein Farbstoff, ein Kuppler, farbig oder farblos kuppelnd, ein Härtungsmittel, ein Silberhalogenidlösungsmittel, ein Verschleierungsmittel, ein Entwicklungsbeschleuniger oder Entwicklungsinhibitor, eine Entwicklerverbindung, ein Bleichinhibitor oder Bleichbeschleuniger, ein Beizmittel oder ein Sensibilisator. Am meisten hervorzuhebende Beispiele für fotografisch wirksame Verbindungen sind Entwicklungsinhibitoren, wie beispielsweise Derivate des Benzotriazols, oder heterocyclische Mercaptoverbindungen wie insbesondere 1-Phenyl-5-mercaptotetrazol (PMT) und Derivate davon.

Y kann unmittelbar den Rest einer fotografisch wirksamen Verbindung darstellen oder aber den Rest einer Vorläuferverbindung davon. Das letztere ist der Fall, wenn die fotografisch wirksame Verbindung bei der Spaltung der Bindung zwischen Y und C zunächst in verkappter Form freigesetzt wird und erst wirksam wird, wenn auch die verkappende Gruppe unter den Bedingungen der fotografischen Entwicklung entfernt wird. So kann z.B. zwischen dem erfindungsgemässen Zeitsteuerglied und dem Rest der eigentlichen fotografisch wirksamen Verbindung ein weiteres Zeitsteuerglied angeordnet sein. In diesem Fall setzt das Wirksamwerden der fotografisch wirksamen Verbindung die Abfolge folgender Vorgänge voraus:

1. Kupplung und Abspaltung des Kupplerrestes von dem erfindungsgemässen Zeitsteuerglied,
2. Abspaltung des erfindungsgemässen Zeitsteuergliedes von dem zusätzlich vorhandenen Zeitsteuerglied, und
3. Abspaltung des zusätzlich vorhandenen Zeitsteuergliedes von der fotografisch wirksamen Verbindung.

Der Rest R ist in zweifacher Hinsicht für das Wirksamwerden der fotografisch wirksamen Verbindung von Bedeutung. Zum einen beeinflusst er im wesentlichen durch seine Grösse die Diffusionsgeschwindigkeit des aus dem Kuppler abgespaltenen Primärproduktes

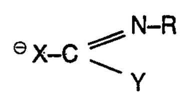 .

Zum anderen ist er im wesentlichen durch seinen Einfluss auf die Elektronenverteilung innerhalb dieser Gruppe massgeblich für die Geschwindigkeit, mit der daraus $Y^{\ominus}$ und damit die fotografisch wirksame Verbindung verfügbar und wirksam wird. Der Rest R ist damit bestimmend für die mittlere Entfernung des Ortes, an dem die fotografisch wirksame Verbindung ihre Wirksamkeit entfaltet, von dem Ort, an dem sich die Vorläuferverbindung befunden hat. Bevorzugt steht R für gegebenenfalls substituiertes Phenyl, wobei durch Variation der Substitution am Phenylring die zeitliche Verzögerung der Freisetzung von Y in gewünschter Weise beeinflusst werden kann.

Eine bei der Definition von R, $R^1$ und $R^2$ erwähnte Alkylgruppe kann geradkettig oder verzweigt sein. Vorzugsweise enthält eine solche Alkylgruppe nicht mehr als 8 C-Atome. Eine bei der Definition von R erwähnte Aralkylgruppe ist beispielsweise Benzyl oder Phenethyl.

Eine bei der Definition von R, $R^1$ und $R^2$ erwähnte Arylgruppe ist vorzugsweise Phenyl, das gegebenenfalls substituiert ist, z.B. mit Halogen, Nitro, $-CF_3$, Alkoxy, Acylamino, Sulfamoyl oder Carbamoyl.

Eine Acylgruppe leitet sich ab durch Entfernen einer Hydroxylgruppe von organischem Carbon- oder Sulfonsäuren, von Kohlensäuremonoestern oder von Carbamin- oder Sulfaminsäuren.

Eine heterocyclische Gruppe ist beispielsweise Benzoxazolyl, Benztriazolyl oder Triazinyl.

Beispiele für erfindungsgemässe Vorläuferverbindungen sind im folgenden aufgeführt:

1)

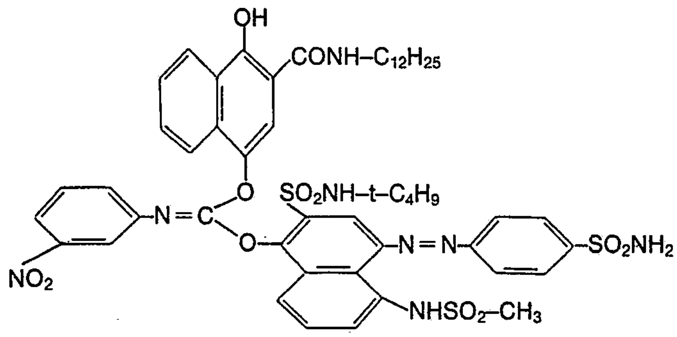

2)

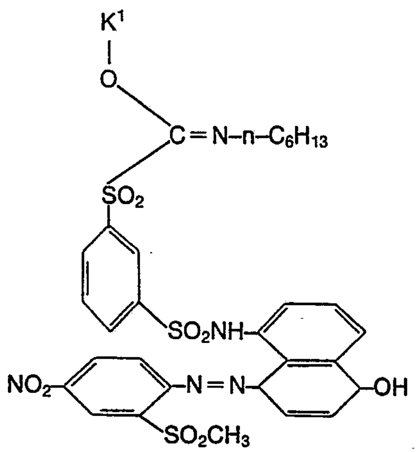

$K^1$ =

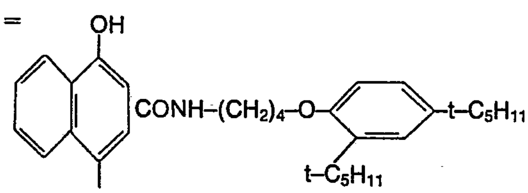

3)

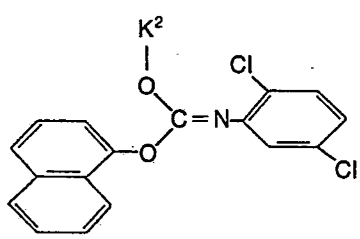

$K_2$ =

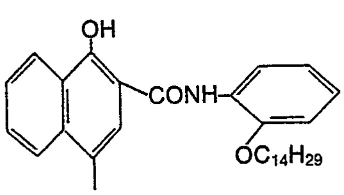

4)

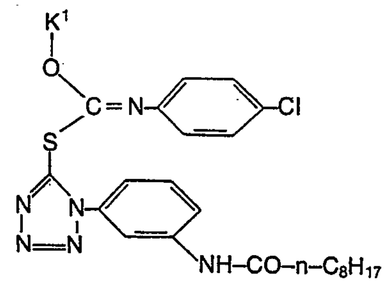

5)

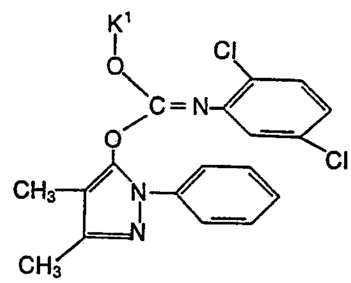

6)

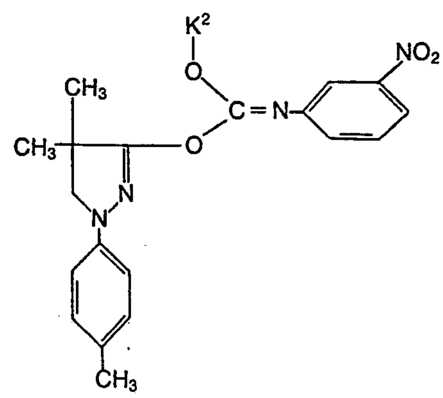

7)

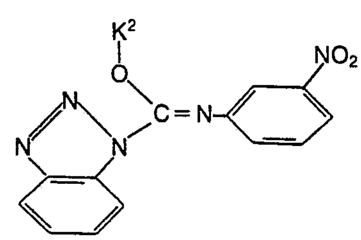

8)

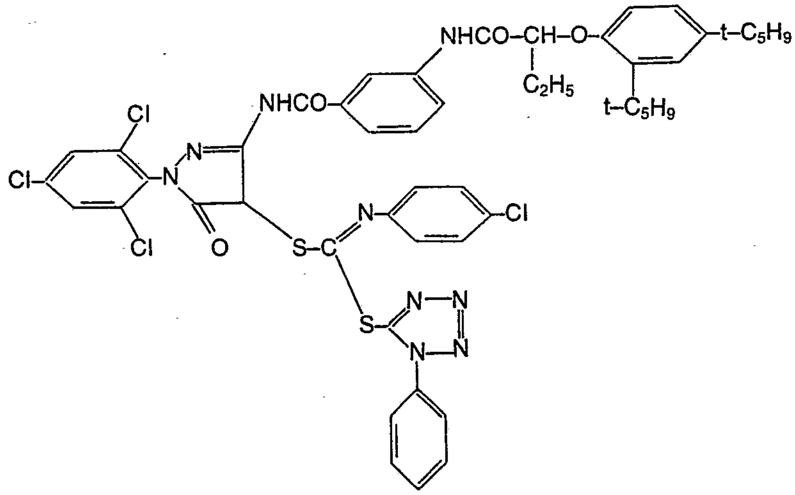

9)

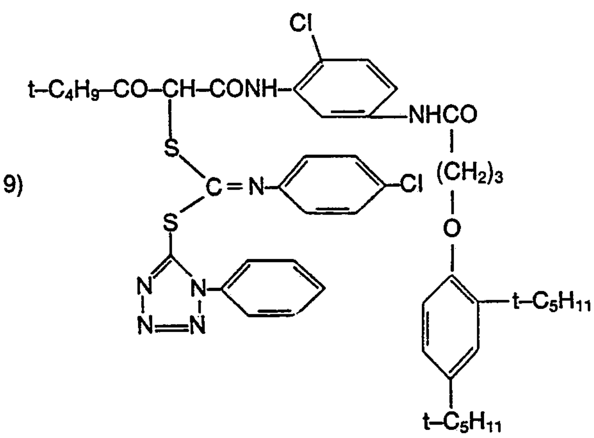

12)

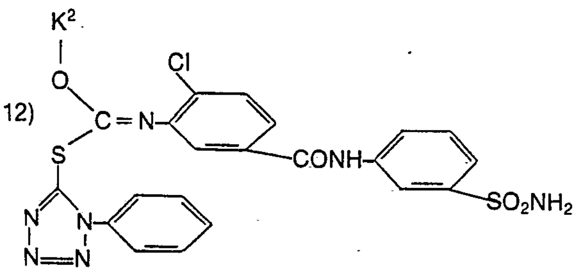

13)

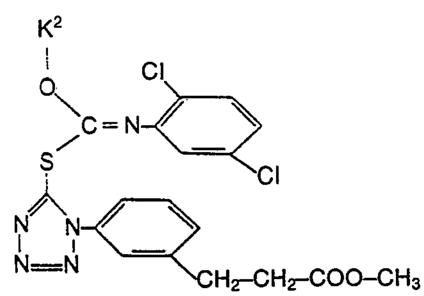

10)

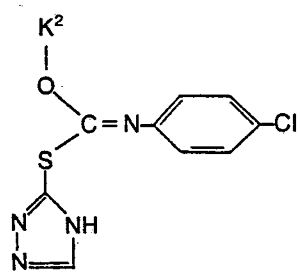

11)

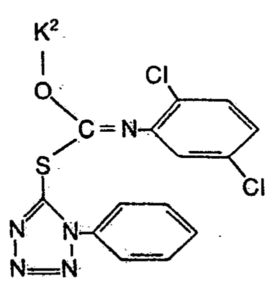

14)

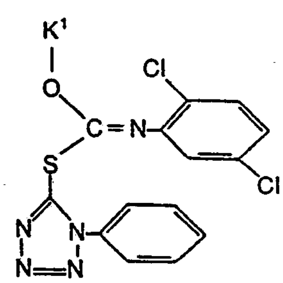

15)

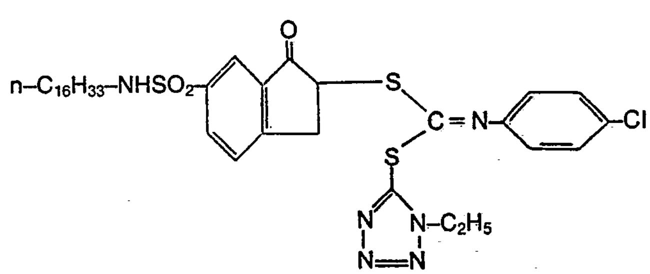

16) 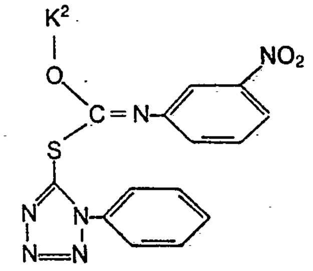

17) 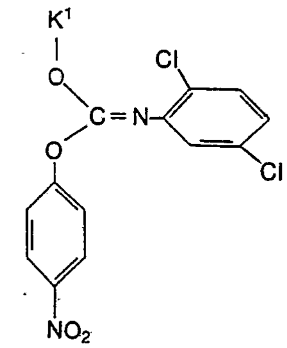

Bei der Freisetzung des an die Kupplungsstelle gebundenen Restes und gegebenenfalls der fotografisch wirksamen Verbindung wird vermutlich folgende Reaktionsfolge durchlaufen:

1) 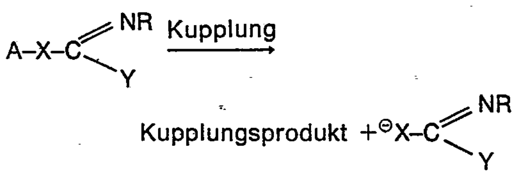


2) 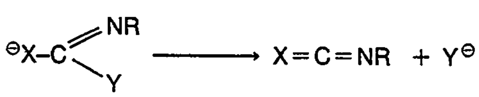

Im ersten Schritt wird das Primärprodukt

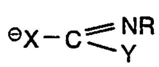

abgespalten, das dann unter den Bedingungen der fotografischen Entwicklung einem Zerfall unterliegt, wobei die fotografisch wirksame Verbindung gegebenenfalls in Form einer alkalilabilen Vorläuferverbindung freigesetzt wird. Die erwähnte alkalilabile Vorläuferverbindung ist zu unterscheiden von den Vorläuferverbindungen, die Gegenstand der vorliegenden Erfindung sind. Letztere sind nicht alkalilabil; sie erfordern für die Freisetzung der fotografisch wirksamen Verbindungen die Anwesenheit von oxidiertem Silberhalogenidentwickler.

Besonders bevorzugt sind solche Verbindungen der Formel I, die als fotografisch wirksame Verbindung einen Entwicklungsinhibitor freisetzen. Bestimmt durch die Lebensdauer und die Diffusionsfähigkeit des aus den erfindungsgemässen Verbindungen freigesetzten Primärproduktes wird eine gesteuerte Verwaschung des Inhibitors in einer gewissen Umgebung des Ortes erreicht, an dem die Kupplung und damit die Freisetzung des Primärproduktes erfolgt. Die Verwaschung des Inhibitors übt bekanntlich einen grossen Einfluss auf den Kanteneffekt und damit auf die Schärfe fotografischer Aufzeichnungsmaterialien aus [Phot. Sci. and Eng. 15, 82 (1971) und 18, 131 (1974)].

Für die Herstellung der erfindungsgemässen Vorläuferverbindungen stehen dem Fachmann verschiedene Methoden zur Verfügung

Methode A:

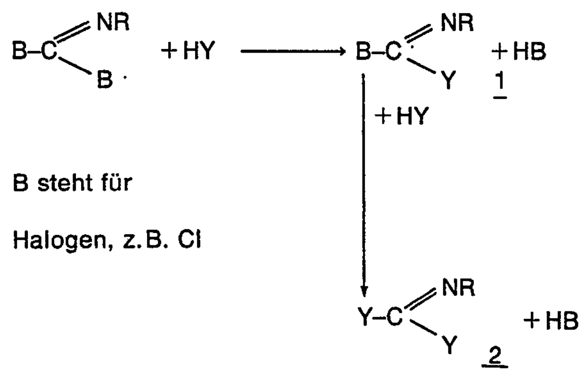


B steht für

Halogen, z.B. Cl

1 oder 2 + A–XH ⟶ I + HB (bzw. HY)
3

Methode B: (für X = O oder S; Y = SR')

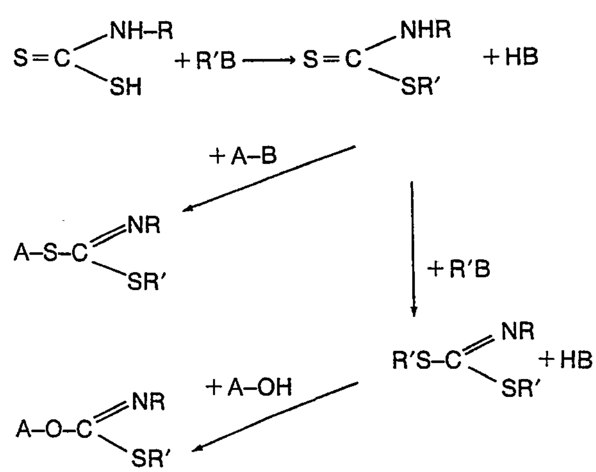


Methode C:

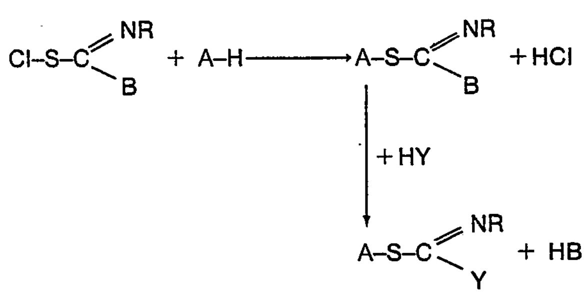

Methode D:

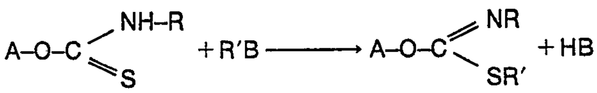

Methode E:

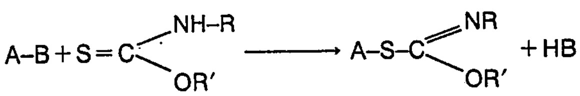

Die aufgeführten Umsetzungen und dabei verwendete Vorprodukte sind aus der Literatur bekannt; z.B.

Methode A:
Angew. Chem. 81, 18 (1969), Synthesis 1982, 984, Synthesis 1971, 575, Chem. Ber. 99, 239 (1966)
Methode B:
J. Med. Chem. 25, 557 (1982), Chem. Ber. 99, 239 (1966)
Methode C:
Synthesis 1970, 561, 575, J. Amer. Chem. Soc. 77, 5171 (1955), Angew. Chem. 77, 427 (1965)
Methode D:
J. Amer. Chem. Soc. 77, 581

Verbindungen der Formel A–OH sind beispielsweise beschrieben in DE-A-2 590 408, US-A-3 419 391 und US-A-3 408 194.

Die Herstellung der Verbindung 11 ist im folgenden beschrieben.

Herstellung der Verbindung 11

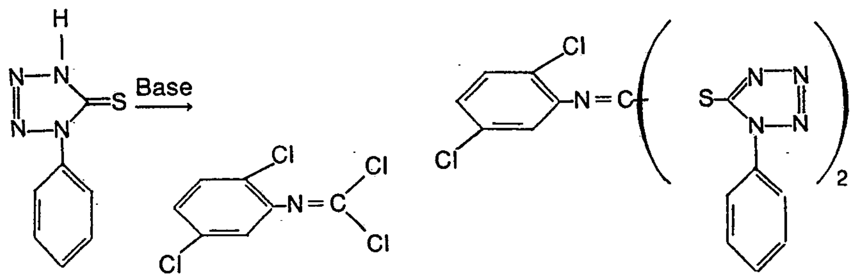


A

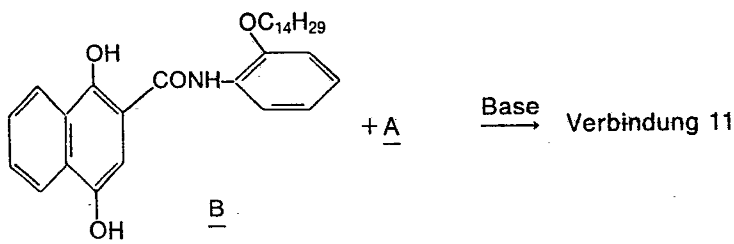


B

Verbindung A (Zwischenprodukt)

| | |
|---|---|
| 17,8 g | 1-Phenyl-5-mercaptotetrazol in |
| 100 ml | Acetonitril suspendiert werden unter Rühren nacheinander mit |
| 12 g | 2,5-Dichlorphenylisocyaniddichlorid und |
| 14 ml | Triethylamin versetzt. |

Nach 1 h werden 80 ml Wasser zugetropft. Der Niederschlag wird gesammelt, mit Wasser und dann mit Methanol gewaschen. Es werden 25 g der Verbindung A erhalten, die sich bei 163 °C zersetzt.

Verbindung 11

Eine Suspension von 5 g 1,4-Dihydroxy-2'-n-tetradecyloxy-2-naphthanilid (Verbindung B) und 5,2 g der Verbindung A in 50 ml Acetonitril werden unter $N_2$ und unter Rühren mit 2 ml Triethylamin versetzt und das Reaktionsgemisch 4 h auf 55 °C erhitzt. Nach Stehen über Nacht bei Raumtemperatur wird der Ansatz mit 160 ml Methanol, denen 2 ml Eisessig zugegeben wurden, unter Rühren versetzt. Es wird noch 1 h im Eisbad gerührt, der Niederschlag abgesaugt und mit Methanol gewaschen.

Der Niederschlag wird in 100 ml Methanol suspendiert und die Suspension 0,5 h nahe dem Siedepunkt gerührt. Nach Erkaltenlassen, zuletzt im Eisbad, werden nach Absaugen und Trocknen 6,9 g (79% d. Theorie) der Verbindung 11 mit Schmelzpunkt 102–103 °C erhalten.

| | Ber. (%) | Gef. (%) |
|---|---|---|
| C | 64,35 | 64,1 |
| H | 5,8 | 5,7 |
| N | 10,0 | 9,8 |

In entsprechender Weise wird die Verbindung 7 erhalten

| | Ber. (%) | Fp 100 °C Gef. (%) |
|---|---|---|
| C | 69,9 | 69,5 |
| H | 6,4 | 6,6 |
| N | 11,1 | 11,1 |

Die erfindungsgemässen Vorläuferverbindungen können in üblichen silberhalogenidhaltigen Aufzeichnungsmaterialien verwendet werden und wirken sich hierbei günstig auf die fotografischen Eigenschaften aus. Bei farbfotografischen Aufzeichnungsmaterialien, insbesondere solchen, die nach dem chromogenen Verfahren entwickelt werden, sind hervorzuhebende Ergebnisse eine deutliche Verbesserung der Empfindlichkeits-Farbkörnigkeits-Relation sowie eine verbesserte Schärfe und Farbwiedergabe aufgrund der erzielten Kanteneffekte und Interimageeffekte, wenn nach vorliegender Erfindung Verbindungen verwendet werden, die einen Entwicklungsinhibitor freisetzen.

Die erfindungsgemässe Vorläuferverbindung ist in mindestens einer Schicht des fotografischen Aufzeichnungsmaterials enthalten, vorzugsweise in einer lichtempfindlichen Silberhalogenidemulsionsschicht. Die Konzentration, in der die erfindungsgemässe Vorläuferverbindung zur Anwendung gelangt, kann je nach Anwendungszweck innerhalb weiter Grenzen variiert werden und liegt beispielsweise für Inhibitor freisetzende Verbindungen bei Anwendung in einer Silberhalogenidschicht zwischen $2 \times 10^{-6}$ und $2 \times 10^{-2}$ Mol pro Mol Silberhalogenid. Die günstigste Konzentration kann vom Fachmann durch routinemässige Reihenversuche leicht ermittelt werden. Die Einarbeitung der erfindungsgemässen Vorläuferverbindungen in die fotografischen Schichten erfolgt nach üblichen Methoden. Günstig ist die Einarbeitung aus Lösungen in organischen Lösungsmittel oder in Form von Emulgaten.

Die Wirkung der aus den erfindungsgemäss verwendeten Vorläuferverbindungen freigesetzten fotografisch wirksamen Verbindungen kann sich sowohl in der Schicht entfalten, in die die Vorläuferverbindungen eingearbeitet sind, als auch in benachbarten Schichten, in die das Primärprodukt bzw. die daraus freigesetzte fotografisch wirksame Verbindung zu diffundieren vermag. Auf diese Weise können mit Hilfe der erfindungsgemässen Vorläuferverbindungen in vielfältiger Weise die bei der Entwicklung in den einzelnen Schichten ablaufenden Vorgänge gesteuert werden, wobei auch unter Ausnutzung der mittels der erfindungsgemässen Verbindungen ermöglichten Nachbareffekte die Entwicklung einer Silberhalogenidemulsionsschicht durch das Ergebnis der bildmässigen Entwicklung in einer anderen Schicht beeinflusst werden kann, so dass man insgesamt eine Verbesserung hinsichtlich Farbkörnigkeit, Schärfe und Farbwiedergabe erzielen kann. Interessant ist auch die Verwendung der erfindungsgemässen Vorläuferverbindungen in Teilschichten einer aus mehreren Teilschichten gleicher Spektralempfindlichkeit bestehenden Teilfarbeneinheit. Solche Teilschichten sind in dem fotografischen Aufzeichnungsmaterial nicht notwendigerweise unmitttelbar übereinander angeordnet, sondern können voneinander durch andere Schichten getrennt sein, z.B. durch eine Teilschicht einer Teilfarbeneinheit anderer Spektralempfindlichkeit.

Schichten unterschiedlicher Spektralempfindlichkeit sind in der Regel durch nicht lichtempfindliche Zwischenschichten voneinander getrennt, um eine unerwünschte Diffusion von Entwickleroxidationsprodukten in nicht (spektral) zugeordnete farbkupplerhaltige Schichten wirksam zu unterbinden. Die Zwischenschichten können reine Bindemittelschichten sein oder Bindemittelschichten, die weitere Zusätze enthalten, z.B. Verbindungen, die mit diffundierenden Entwickleroxidationsprodukten zu reagieren vermögen, Filterfarbstoffe, Härtungsmittel, eingelagerte Entwickler oder UV-Absorber.

Sofern das erfindungsgemässe fotografische Aufzeichnungsmaterial Farbkuppler enthält, handelt es sich hierbei im allgemeinen um solche Kuppler, die mit Hilfe von Ölbildnern in fotografische Giesslösungen eingearbeitet werden können. Dies sind in der Regel Kuppler, die in organischen Lösungsmitteln, insbesondere in Ölbildnern löslich sind (sogenannte hydrophobe Kuppler). Es können jedoch auch hydrophile Kuppler verwendet werden. Die Kuppler sind 2-, 4- oder 6-Äquivalent-Farbkuppler (Research Disclosure 19536 – Juli 1980). Sie sind farblos oder farbig (Maskenkuppler) und enthalten üblicherweise, vorzugsweise in einer nicht kuppelnden Stellung eine Ballastgruppe, so dass sie in diffusionsfester Form in der jeweiligen Schicht eingelagert sind. Bei der chromogenen Entwicklung entstehen aus ihnen die verschiedenen Bildfarbstoffe. Zusätzlich können weitere Kuppler verwendet werden, z.B. Weisskuppler oder Kuppler, die bildmässig eine fotografisch wirksame Gruppe freisetzen.

Für die Entwicklung der erfindungsgemässen fotografischen Aufzeichnungsmaterialien werden bevorzugt solche Entwickler verwendet, wie sie üblicherweise bei der chromogenen Entwicklung zur Anwendung gelangen. Beispiele sind die bekannten p-Phenylendiaminderivate wie
N,N-Dimethyl-p-phenylendiamin,
N,N-Diethyl-p-phenylendiamin,
Monomethyl-p-phenylendiamin,
2-Amino-5-diethylaminotoluol,
N-Butyl-N-ω-sulfobutyl-p-phenylendiamin,
2-Amino-5-(N-ethyl-N-β-methansulfonamidoethylamino)-toluol,
N-Ethyl-N-β-hydroxyethyl-p-phenylendiamin,
N,N-bis-(β-hydroxyethyl)-p-phenylendiamin,
2-Amino-5-(N-ethyl-N-β-hydroxyethylamino)-toluol und dergl.

Weitere brauchbare Farbentwickler sind beispielsweise beschrieben in J. Amer. Chem. Soc. 73, 3100 (1951).

Für die Freisetzung der fotografisch wirksamen Verbindung aus der erfindungsgemässen Vorläuferverbindung kommt es bei der Auswahl einer geeigneten Silberhalogenidentwicklerverbindung hauptsächlich auf deren Fähigkeit an mit der Vorläuferverbindung eine oxidative Kupplung einzugehen. Von geringerer Bedeutung ist die Qualität des hierbei erzeugten Farbstoffes. Es können daher auch solche Silberhalogenidentwicklerverbindungen verwendet werden, die bei der Kupplung entweder keine stabilen Farbstoffe bilden oder

Farbstoffe, die in bezug auf ihre Farbstoffeigenschaften nicht optimal sind.

Beispiel 1

500 ml einer schwefel- und gold-gereiften, rotsensibilisierten Silberbromidiodidemulsion (6 Mol-% Iodid; mittlerer Korndurchmesser 0,65 µm), die pro kg die 200 g $AgNO_3$ äquivalente Menge an Silberhalogenid und 50 g Gelatine enthielt und mit 1 g 4-Hydroxy-6-methyl-1,3,3a,7-tetraazainden stabilisiert war, wurde mit einem der in der folgenden Tabelle 1 angegebenen DIR-Kuppler versetzt und mit einem Farbkuppleremulgat aus 25 g Farbkuppler der folgenden Formel

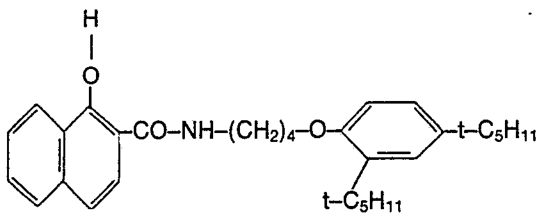

25 g Trikresylphosphat und 25 g Gelatine abgemischt.

Der DIR-Kuppler war zusammen mit Trikresylphosphat und Gelatine im Gewichtsverhältnis 1:1:1 emulgiert. Es wurden jeweils $3 \times 10^{-3}$ Mol des DIR-Kupplers auf 1 Mol Silberhalogenid gegeben.

Ausserdem wurde in der gleichen Weise eine Probe (Emulsion + Farbkuppleremulgat) ohne DIR-Kupplerzusatz hergestellt.

Die auf diese Weise erhaltenen Giesslösungen wurden auf Schichtträger vergossen (Silberauftrag: 2,0 g pro $m^2$) und gehärtet und ergaben die Materialien 1 bis 6 (vergl. Tabelle 1).

Die Grösse des «Kanten-Effekts» wurde wie bei T.H. James, The Theory of the Photographic Process, 4. Aufl. Macmillan Publ. Co. Inc. New York/London (1977) S. 609–614 beschrieben (zur Vermeidung der Lichtstreuung) mittels Röntgenbestrahlung bestimmt: Und zwar wurde auf die Proben jeweils mit gleicher Röntgendosis sowohl ein Makrofeld als auch ein 30 µm breiter Spalt aufbelichtet. Danach wurden die Proben verarbeitet nach einem Color-Negativ Verfahren, das in «The British Journal of Photography», 1974, Seiten 597 und 598 beschrieben ist. Die an diesen Proben dann gemessene Dichtedifferenz zwischen Spalt (= Mikrodichte) und dem Makrofeld (= Makrodichte) bei derjenigen Röntgendosis, welche die Makrodichte = 1,0 ergibt, dient in Tabelle 1 als Mass für die Grösse des Kanten-Effekts.

Tabelle 1

| Material | DIR-Kuppler | Kanteneffekt bei Makrodichte = 1 bg |
|---|---|---|
| 1 | – | 0,05 |
| 2 | A | 0,32 |
| 3 | B | 0,42 |
| 4 | 11 | 0,52 |
| 5 | 14 | 0,50 |
| 6 | 16 | 0,49 |

Beispiel 2

Ein farbfotografisches Color-Negativ-Aufzeichnungsmaterial (Material 7) wurde hergestellt, in dem auf einen transparenten Schichtträger aus Cellulosetriacetat die folgenden Schichten in der angegebenen Reihenfolge aufgetragen wurden. Die Mengenangaben beziehen sich jeweils auf 1 $m^2$. Für den Silberhalogenidauftrag werden die entsprechenden Mengen $AgNO_3$ angegeben.

Schicht 1 (Antihaloschicht)

Schwarzes kolloidales Silbersol mit 1,5 g Gelatine und 0,33 g Ag

Schicht 2 (Zwischenschicht)

0,6 g Gelatine

Schicht 3 (1. rotsensibilisierte Schicht)

rotsensibilisierte Silberbromidiodidemulsion (5,5 Mol-% Iodid; mittlerer Korndurchmesser 0,45 µm) mit 3,8 g $AgNO_3$, 2,5 g Gelatine, 0,9 g Blaugrünkuppler der folgenden Formel:

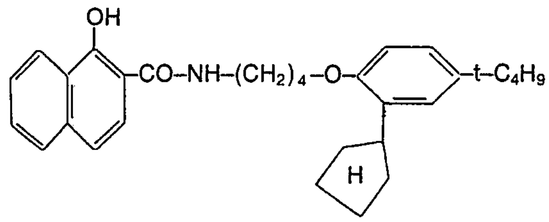

und 0,1 g Maskenkuppler der folgenden Formel

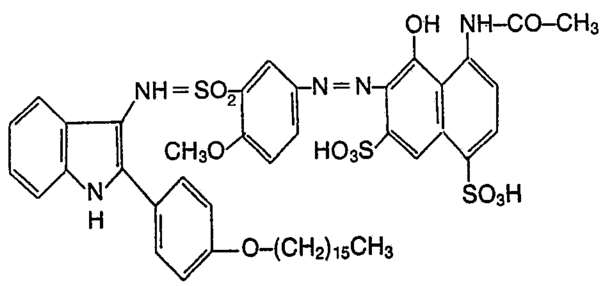

Schicht 4 (2. rotsensibilisierte Schicht)

rotsensibilisierte Silberbromidiodidemulsion (6,5 Mol-% Iodid; mittlerer Korndurchmesser 0,8 µm) mit 3,0 g $AgNO_3$, 2,0 g Gelatine und 0,2 g des in Schicht 3 enthaltenen Blaugrünkupplers.

Schicht 5 (Zwischenschicht)

0,7 g Gelatine und 0,2 g-Diisooctylhydrochinon

Schicht 6 (1. grünsensibilisierte Schicht)

grünsensibilisierte Silberbromidiodidemulsion (4,8 Mol-% Iodid; mittlerer Korndurchmesser 0,40 µm) mit 3,0 g $AgNO_3$, 2,4 g Gelatine und 0,9 g Purpurkuppler der folgenden Formel

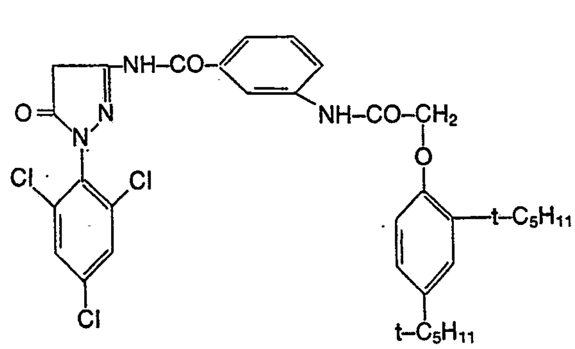

0,09 g DIR-Kuppler der Formel

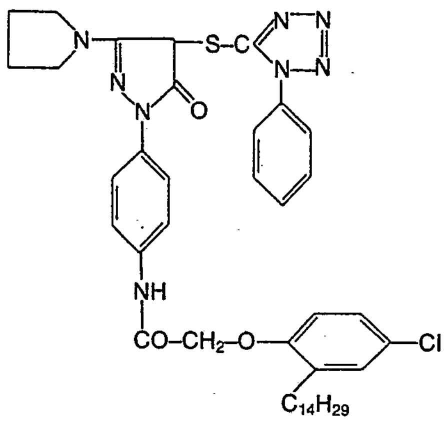

und 0,1 g Maskenkuppler der Formel

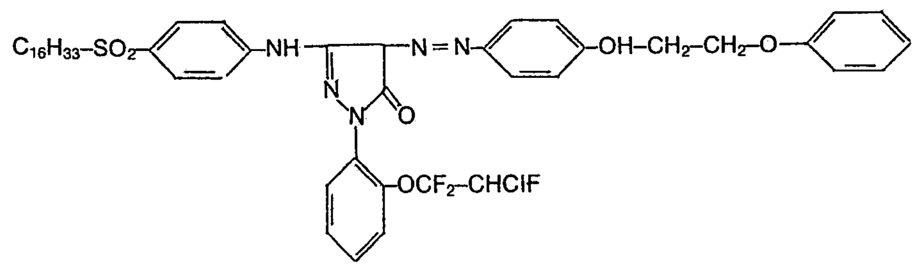

Schicht 7 (2. grünsensibilisierte Schicht)

grünsensibilisierte Silberbromidiodidemulsion (4,3 Mol-% Iodid; mittlerer Korndurchmesser 0,70 µm) mit 2,5 g $AgNO_3$, 1,6 g Gelatine und 0,21 g des in Schicht 6 enthaltenen Purpurkupplers sowie 0,02 g des in Schicht 6 enthaltenen Maskenkupplers.

Schicht 8 (Zwischenschicht)

0,5 g Gelatine und 0,15 g 2,5-Diisooctylhydrochinon

Schicht 9 (Gelbfilterschicht)

gelbes kolloidales Silbersol mit 0,2 g Ag und 0,9 g Gelatine

Schicht 10 (1. blauempfindliche Schicht)

blausensibilisierte Silberbromidiodidemulsion (4,9 Mol-% Iodid; mittlerer Korndurchmesser 0,45 µm) mit 0,76 g $AgNO_3$, 0,85 g Gelatine und 1,35 g Gelbkuppler der folgenden Formel

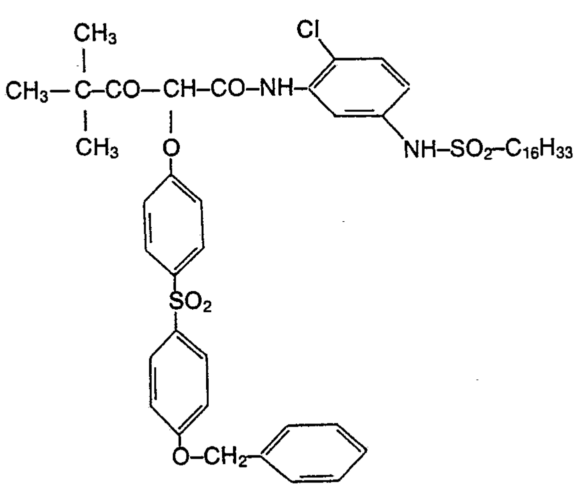

Schicht 11 (2. blauempfindliche Schicht)

blausensibilisierte Silberbromidiodidemulsion (3,3 Mol-% Iodid; mittlerer Korndurchmesser 0,85 µm) mit 1,0 g $AgNO_3$, 0,85 g Gelatine und 0,5 g des in Schicht 10 enthaltenen Gelbkupplers

Schicht 12 (Schutzschicht)

1,2 g Gelatine

Schicht 13 (Härtungsschicht)

1,5 g Gelatine und 0,7 g Härtungsmittel der folgenden Formel

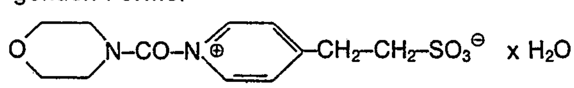

Alle Silberhalogenidemulsionen dieses Materials waren mit 0,5 g 4-Hydroxy-6-methyl-1,3,3a,7-tetraazainden pro 100 g $AgNO_3$ stabilisiert.

Weitere Materialien 8 bis 12 wurden in entsprechender Weise hergestellt, wobei jedoch in der Schicht 3 zusätzlich 0,11 mMol eines der in der folgenden Tabelle 2 aufgeführten DIR-Kuppler enthalten waren.

An den Materialien 7 bis 12 wurde wie in Beispiel 1 erläutert die Grösse der Kanteneffekte ermittelt. Aus Tabelle 2 ist ersichtlich, dass durch die in die Schicht 3 eingelagerten erfindungsgemässen DIR-Kuppler nicht nur im Blaugrün, sondern (offenbar durch vertikale Nachbareffekte) auch im Purpur höhere Kanteneffekte erzielt werden.

Ausserdem wurde an den Materialien 7 bis 12 die Schärfe mit Hilfe der Modulations-Transferfunktion (MTF) bestimmt. Die Methode ist beschrieben bei T.H. James, The Theory of the Photographic Process, 4. Aufl., Macmillan Publ. Co. Inc. New York/London (1977) S. 605.

In Tabelle 2 sind für die Materialien 7 bis 12 diejenigen Ortsfrequenzen (in Linien pro mm) eingetragen, bei denen die MTF einen Wert von 50% hat. Aus den höheren MTF-Werten bei den erfindungsgemässen DIR-Kupplern erkennt man, dass diese eine höhere Bildschärfe liefern.

Auch der die Farbqualität verbessernde Interimage-Effekt in der Wirkung von Blaugrün nach Purpur wird durch die erfindungsgemässe DIR-Kuppler verbessert. Als «Purpur-Interimage-Effekt» ($IIE_{pp}$) ist in Tabelle 2 eingetragen, um wieviel Prozent die Purpurgradation bei Grünbelichtung grösser ist als bei Weissbelichtung.

Die Verarbeitung der in diesem Beispiel angegebenen Materialien erfolgt bei Bestimmung des Kanteneffekts, der MTF und des Interimage-Effekts nach dem gleichen Color-Negativ-Verfahren wie bei Beispiel 1.

Tabelle 2

| Material | DIR-Kuppler | Purpur-Interimageeffekt [%] | Kanteneffekt bei Makrodichte = 1 | | MTF = 50% Linien/mm | |
|---|---|---|---|---|---|---|
| | | | blaugrün | purpur | blaugrün | purpur |
| 7 | – | −15 | 0,15 | 0,10 | 20 | 32 |
| 8 | A | +10 | 0,35 | 0,26 | 24 | 35 |
| 9 | B | +20 | 0,40 | 0,30 | 28 | 38 |
| 10 | 11 | +25 | 0,48 | 0,35 | 31 | 40 |
| 11 | 14 | +25 | 0,48 | 0,34 | 30 | 38 |
| 12 | 16 | +20 | 0,45 | 0,34 | 29 | 38 |

Zum Vergleich wurden (in den Materialien 2, 3, 8 und 9) die folgenden nicht erfindungsgemässen DIR-Kuppler verwendet

DIR-Kuppler A

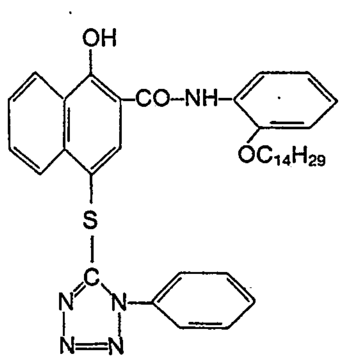

DIR-Kuppler B

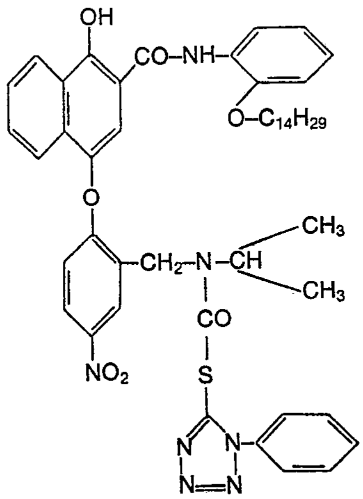

= Kuppler 5 aus US-A-4 248 962

**Patentansprüche**

1. Fotografisches Aufzeichnungsmaterial mit mindestens einer Silberhalogenidemulsionsschicht, gekennzeichnet durch den Gehalt an einer Verbindung der folgenden Formel I

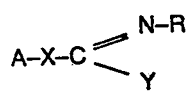

I

worin bedeuten

A den Rest einer Verbindung, die unter den Bedingungen der fotografischen Entwicklung zur oxidativen Kupplung mit einer Silberhalogenidentwicklerverbindung befähigt ist;

X –O–, –S– oder $-NR^1-$;

Y einen abspaltbaren Rest, der unter den Bedingungen der fotografischen Entwicklung unter Mitnahme des Bindungselektronenpaares zwischen C und Y von C abgespalten wird, wenn X unter Mitnahme des Bindungselektronenpaares zwischen A und X von A abgespalten wird, und der der Rest einer fotografisch wirksamen Verbindung oder der Rest einer alkalilabilen Vorläuferverbindung einer fotografisch wirksamen Verbindung ist;

R Alkyl, Aralkyl, Aryl, Acyl, eine heterocyclische Gruppe oder eine Gruppe –NH-Acyl, $-OR^2$ oder $-P(O)(OR^2)_2$;

$R^1$ H, Alkyl, Aryl, Alkylsulfonyl oder Arylsulfonyl; und

$R^2$ Alkyl, Aryl oder eine heterocyclische Gruppe.

2. Aufzeichnungsmaterial nach Anspruch 1, dadurch gekennzeichnet, dass in Formel I der abspaltbare Rest Y über ein Sauerstoff-, Schwefel- oder Stickstoffatom an das in Formel I dargestellte Kohlenstoffatom gebunden ist.

3. Aufzeichnungsmaterial nach Anspruch 1, dadurch gekennzeichnet, dass in Formel I R einen

gegebenenfalls mit Halogen, Nitro, $CF_3$, Alkoxy, Acylamino, Sulfamoyl und/oder Carbamoyl substituierten Phenylring bedeutet.

4. Aufzeichnungsmaterial nach Anspruch 1, dadurch gekennzeichnet, dass in Formel I Y den Rest eines Entwicklungsinhibitors darstellt.

## Claims

1. Photographic recording material containing at least one silver halide emulsion layer, characterised by containing a compound corresponding to the following formula I:

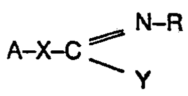

I

wherein

A denotes the residue of a compound which is capable of oxidative coupling with a silver halide developer compound under the conditions of photographic development;

X denotes –O–, –S– or $-NR^1-$;

Y denotes a removable group which is split off from C under the conditions of photographic development, taking with it the linkage electron pair between C and Y when X is split off from A, taking with it the linkage electron pair between A and X, the group denoted by Y being the residue of a photographically active compound or the residue of an alkali-labile precursor compound of a photographically active compound;

R denotes alkyl, aralkyl, aryl, acyl, a heterocyclic group or a group –NH-acyl, $-OR^2$ or $-P(O)(OR^2)_2$;

$R^1$ denotes H, alkyl, alkylsulphonyl or arylsulphonyl and

$R^2$ denotes alkyl, aryl or a heterocyclic group.

2. Recording material according to claim 1, characterised in that in formula I, the removable group Y is attached to the carbon atom of Formula I by an oxygen, sulphur or nitrogen atom.

3. Recording material according to claim 1, characterised in that in formula I, R stands for a phenyl ring optionally substituted with halogen, nitro, $CF_3$, alkoxy, acylamino, sulphoamoyl and/or carbamoyl.

4. Recording material according to claim 1, characterised in that in Formula I, Y stands for the residue of a development inhibitor.

## Revendications

1. Matière de support pour enregistrement photographique comportant au moins une couche d'émulsion d'halogénure d'argent, matière caractérisée en ce qu'elle contient un composé répondant à la formule I suivante:

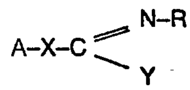

I

dans laquelle

A représente le reste d'un composé qui, dans les conditions du développement photographique, est capable d'une copulation oxydante avec un composé de développement, ou révélateur, pour halogénure d'argent;

X représente –O–, –S– ou $-NR^1-$;

Y représente un reste séparable et qui, dans les conditions du développement photographique, est séparé de C en entraînant avec lui la paire des électrons de liaison entre C et Y, quand X est séparé de A en entraînant avec lui la paire des électrons de liaison entre A et X, et le reste d'un composé actif du point de vue photographique ou le reste d'un précurseur, labile en milieu alcalin, d'un composé actif du point de vue photographique;

R représente un groupe alkyle, aralkyle, aryle, acyle, un groupe hétérocyclique ou un groupe –NH-acyle, $-OR^2$ ou $-P(O)(OR^2)_2$;

$R^1$ représente H, un groupe alkyle, aryle, alkylsulfonyle ou arylsulfonyle; et

$R^2$ représente un groupe alkyle, aryle ou un groupe hétérocyclique.

2. Matière de support pour enregistrement selon la revendication 1, caractérisée en ce que, dans la formule I, le reste séparable Y est relié par l'intermédiaire d'un atome d'oxygène, de soufre ou d'azote à l'atome de carbone représenté sur la formule I.

3. Matière de support pour enregistrement selon la revendication 1, caractérisée en ce que, dans la formule I, R représente un noyau phénylique, éventuellement substitué par de l'halogène, par un reste nitro, $CF_3$, alcoxy, acylamino, sulfamoyle et/ou carbamoyle.

4. Matière de support pour enregistrement selon la revendication 1, caractérisée en ce que, dans la formule I, Y représente le reste d'un inhibiteur du développement.